# EUROPEAN PATENT SPECIFICATION

(11) **EP 1 888 054 B1**
(45) Date of publication and mention of the grant of the patent: **23.03.2011**
(21) Application number: 06770609.3
(22) Date of filing: 18.05.2006
(51) Int. Cl.: A61K 31/16, A61K 31/10, A61P 31/00

(54) **ANTIMICROBIAL COMPOSITION**
ANTIMIKROBIELLE ZUSAMMENSETZUNG
COMPOSITION ANTIMICROBIENNE

(30) Priority: 19.05.2005 US 133007
(43) Date of publication of application: 20.02.2008
(73) Proprietor: ETHICON, INC., Somerville, New Jersey 08876-0151 (US)
(72) Inventor: MING, Xintian, Bridgewater, New Jersey 08807 (US); ROTHENBURGER, Stephen J., Neshanic Station, New Jersey 08853 (US)
(74) Representative: James, Anthony Christopher W.P.
(86) International application number: PCT/US2006/019332
(87) International publication number: WO 2006/125121

(56) References cited:
- WO-A-03/043593
- US-A- 5 283 264
- US-B1- 6 216 699

## Description

### FIELD OF INVENTION

This invention relates to a novel antimicrobial composition comprising (a) a cationic surfactant derived from the condensation of fatty acids and esterified dibasic amino acids, and (b) an iodinated sulfone compound; and to methods of preparation and uses thereof. More specifically, this invention relates to a novel antimicrobial composition comprising lauric arginate (LAE) and diiodomethyl-p-tolylsulfone (DIMPTS). The present invention also relates to medical devices utilizing such novel antimicrobial compositions.

### BACKGROUND OF THE INVENTION

Each year, patients undergo a vast number of surgical procedures in the United States. Current data shows about twenty-seven million procedures are performed per year. Post-operative or surgical site infections ("SSIs") occur in approximately two to three percent of all cases. This amounts to more than 675,000 SSIs each year.

Whenever a medical device is used in a surgical setting, a risk of infection is created. The risk of infection dramatically increases for invasive or implantable medical devices, such as intravenous catheters, arterial grafts, intrathecal or intracerebral shunts and prosthetic devices, which create a portal of entry for pathogens while in intimate contact with body tissues and fluids. The occurrence of SSIs is often associated with bacteria that colonize on the medical device. For example, during a surgical procedure, bacteria from the surrounding atmosphere may enter the surgical site and attach to the medical device. Bacteria can use the implanted medical device as a pathway to surrounding tissue. Such bacterial colonization on the medical device may lead to infection and morbidity and mortality to the patient.

A number of methods for reducing the risk of infection associated with invasive or implantable medical devices have been developed that incorporate antimicrobial agents into the medical devices. Such devices desirably provide effective levels of antimicrobial agent while the device is being used. However, medical devices containing a single antimicrobial agent can suffer loss of efficacy resulting from low concentrations of the agent on the device.

One potential solution to this problem is to use a combination of antimicrobial agents that requires relatively low concentrations of the individual antimicrobial agents. In particular, it is beneficial if the individual antimicrobial agents have differing patterns of bioavailability and different modes of action. The use of a blend of antimicrobial agents is often desirable to achieve a broader spectrum of antimicrobial activity against the various organisms, at reduced levels of the individual antimicrobial agents.

WO 2003/043593 describes an antimicrobial system that uses a cationic surfactant derived from the condensation of fatty acids and esterified dibasic amino acids, as an antimicrobial activity enhancer, in combination with common antimicrobial agents. In particular, this reference describes the use of lauric arginate (LAE) in combination with 2,4, 4'-trichloro-2'-hydroxydiphenylether (triclosan), 3,4,4- trichlorocarbanilid (triclocarban), 2-phenoxyethanol, chlorhexidine salts, hexetidine and cetylpyridinium salts, for cosmetic formulations and preparations directed to avoid body odour and to provide oral care.

The use of diiodomethyl-p-tolylsulfone as an antimicrobial agent is desirable because it is a broad spectrum antimicrobial agent. For example, blends of diiodomethyl-p-tolylsulfone, such as Amical-48 (commercially available from Dow), and acrylic hot melt adhesive polymers have been reported in US 6,216,699, for use in surgical incise drapes having antimicrobial properties. Such blends were reported to indicate zones of inhibition against several organisms including *S*. *aureus, E. coli, P. aeruginosa, K. pneumoniae, P. cepacia, E. cloacae, S. marcescens, S.pyogenes, E. faecalis-Vancomycin Resistant, C. albicans and B. subtilis.*

US-A-5283264 describes antimicrobial compositions comprising diiodomethyl p-tolyl sulfone, N-methylpyrrolidone, water and a surfactant.

There have been no reports to date on the use of a combination of (a) a cationic surfactant derived from the condensation of fatty acids and esterified dibasic amino acids, and (b) iodinated sulfone compound, which has been discovered to exhibit a synergistic effect when, for example, LAE and an iodinated sulfone compound are used in combination, resulting in an enhanced antimicrobial activity against a broader spectrum of the organisms,

### SUMMARY OF THE INVENTION

Described herein is an antimicrobial composition comprising (a) a cationic surfactant derived from the condensation of fatty acids and esterified dibasic amino acids, such as lauric arginate, and (b) an iodinated sulfone compound such as diiodomethyl-p-tolylsulfone. The composition may be used as a stand-alone antimicrobial formulation, or in combination with medical articles or medical devices.

### DETAILED DESCRIPTION OF THE INVENTION

The present invention is directed to an antimicrobial composition comprising (a) a cationic surfactant derived from the condensation of fatty acids and esterified dibasic amino acids, and (b) an iodinated sulfone compound. More specifically, the present invention is directed to an antimicrobial composition comprising lauric arginate (LAE) and diiodomethyl-p-tolylsulfone (DIMPTS), that may be used alone or in combination with medical devices, wherein the antimicrobial properties of the device are improved by incorporation of lauric arginate (LAE) and diiodomethyl-p-tolylsulfone (DIMPTS) therein.

The cationic surfactant described herein is derived from the condensation of fatty acids and esterified dibasic amino acids. More particularly, the cationic surfactant refers to a class of compounds derived from the condensation of fatty acids and esterified dibasic amino acids having the following formula: 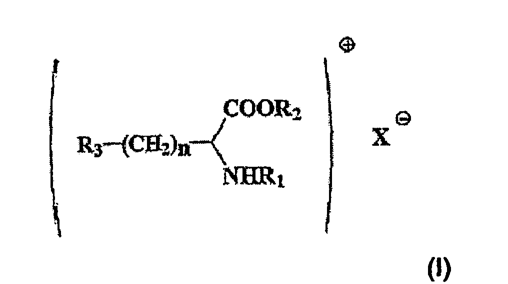 where X is Br, Cl, or HSO₄; R₁ is a linear alkyl chain from a saturated fatty acid or hydroxyacid having from 8 to 14 carbon atoms bonded to the alpha-amino acid group through an amidic bond; R₂ is a linear or branched alkyl chain from 1 to 18 carbon atoms or an aromatic group; R₃ is one of the following:

-NH₃

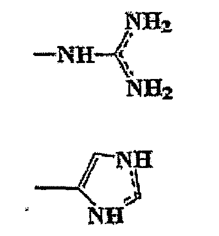

and n can be from 0 to 4.

A particular example of such class of cationic surfactant is lauric arginate (LAE - manufactured by Lamirsa Laboratories, Barcelona, Spain). Lauric arginate, a cationic preservative derived from lauric acid and arginine, in particular, the ethyl ester of the lauramide of the arginine monohydrochloride, can be used to protect against the growth of microorganisms. The chemical structure of LAE is described in formula (III):

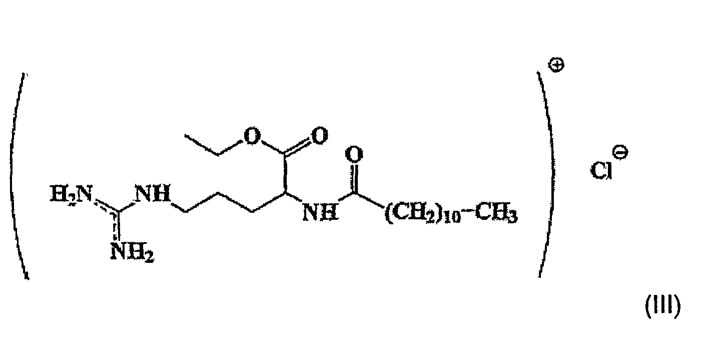

The iodinated sulfone compound used herein refers to an iodinated sulfonic aliphatic or aromatic compound. An example of such compound is diiodomethyl-p-tolylsulfone.

In one particular set of non-limiting embodiments, the antimicrobial composition comprises (a) a cationic surfactant derived from the condensation of fatty acids and esterified dibasic amino acids and (b) an iodinated sulfone compound, as a stand-alone antimicrobial composition formulation, independent of any medical devices or specific applications. Formulation of the antimicrobial composition according to the present invention may be of liquid (e.g. solutions) or solid form (e.g. powders). For instance, the antimicrobial composition may be applied directly to a wound. In another set of non-limiting embodiments, the present invention provides medical devices incorporated with the antimicrobial composition. The terms "incorporate", "incorporated", or "incorporating", as used herein, refer to combining the composition with the medical device by physical or chemical means. Examples include, but are not limited to, impregnating, dipping, soaking or coating a medical device with the antimicrobial composition or preparing the medical device by adding the antimicrobial composition to the material that the medical device is made from.

The medical devices that may be treated according to the invention are either fabricated from or coated or treated with a biomedical polymer and include, but are not limited to, microcapsules, dressings, implants, wound closures, staples, meshes, controlled drug delivery systems, wound coverings, fillers, sutures, tissue adhesives, tissue sealants, absorbable and non-absorbable hemostats, catheters including urinary catheters and vascular catheters (e.g., peripheral and central vascular catheters), wound drainage tubes, arterial grafts, soft tissue patches (such as polytetrafluoroethylene ("PTFE") soft tissue patches), gloves, shunts, stents, tracheal catheters, wound dressings, sutures, guide wires and prosthetic devices (e.g., heart valves and LVADs). The present invention may be further applied to medical articles that have been prepared according to U.S. Patent Nos. 3,839,297; 4,027,676; 4,185,637 and 4,201,216.

In further and various non-limiting embodiments, the present invention provides for a stand alone antimicrobial composition comprising LAE in an amount from 0.001% to 10% by weight based on the total weight of the composition and the concentration of the iodinated sulfone compound ranges from 0.001% to 5 % by weight relative to total weight of the composition. More preferably, the antimicrobial composition of the invention comprises LAE in particular an amount from 0.01% to 2% by weight based on the total weight of the composition and the concentration of the iodinated sulfone compound from 0.01% to 1.5% by weight relative to total weight of the composition. In particular non-limiting embodiments of the invention, where the medical article is a suture, such as USP 2-0 polyglactin 910 sutures, the amount of LAE contained is about 0.001-100 µg/cm, preferably about 0.01-50 µg/cm and the amount of diiodomethyl-p-tolylsulfone is 0.00.1 to 100 µg/cm, preferably 0.01 to 50 µg/cm. Additionally, the invention provides for medical devices incorporated with the antimicrobial composition as described herein. The incorporation of LAE and an antimicrobial metal will enhance the antimicrobial activity of the device against a broader spectrum of pathogens.

It has been found that the combination of LAE with diiodomethyl-p-tolylsulfone have better activity than LAE alone or the diiodomethyl-p-tolylsulfone alone, and that LAE functions as an antimicrobial enhancing agent. This enhancement of activity by LAE may be explained by its mode of action that damages the cytoplasmatic membrane of the microorganisms. LAE's metabolism in rats has been studied showing a fast absorption and metabolism into naturally-occurring amino acids and the lauric acid, which are eventually excreted as carbon dioxide and urea. Toxicological studies have demonstrated LAE is completely harmless to animals and humans.

The antimicrobial composition according to the present invention is characterized by its synergistic activity. It has now been found that the antimicrobial activity of the combination of LAE with the iodinated sulfone compound is higher than the activity displayed by each of the components when used individually at the same dosage. This enhanced antimicrobial efficacy allows the composition to have potent efficacy against a wide range of microorganisms at levels where the two compounds used individually would not be as effective. The use of this combination has been shown to enhance the antimicrobial spectrum against several organisms including, but not limited, *Tinea pedis, Tinea unguium, Tinea cruris,* or *Tinea capitis, S. aureus" MRSA, MRSE, GISA, S. epidermidis, E. coli, P. aeruginosa, K. pneumoniae, B. cepacia, E. cloacae, S. marcescens, S. pyogenes, S. agalacticae, E. faecalis-Vancomycin Resistant, E faecium, C. albicans and B. subtilis, Salmonella sp., Proteus sp., Acinetobacter sp.Aspergillus niger.*

The term "effective antimicrobial activity" refers to an ability to decrease the number of colony-forming units of a bacterium or yeast, in a defined period of time, by a factor of ten or more and preferably a factor of 100 or more.

In an alternative embodiment, surgical articles including sutures may be coated with LAE or DIMPTS individually, in an effective antimicrobial amount.

While the following examples demonstrate certain embodiments of the invention, they are not to be interpreted as limiting the scope of the invention, but rather as contributing to a complete description of the invention.

### Example 1

### Antimicrobial Efficacy of LAE- diiodomethyl-p-tolylsulfone antimicrobial composition in vitro

The synergistic antimicrobial efficacy of the LAE and diiodomethyl-p-tolylsulfone (DIMPTS) antimicrobial composition is illustrated by the results shown in Tables 1 and 2, and was determined by the following protocol. Lauric arginate (LAE) and diiodomethyl-p-tolylsulfone (DIMPTS) stock solutions were prepared at the concentration of 1000ppm in sterile saline and ethanol respectively. Sequential dilutions of the above two stock solutions were then prepared in sterile saline. A portion of 0.05 ml of each dilution was added to 0.95 ml of bacterial culture (trypticase soy broth containing 10⁶ CFU/ml). Controls contained similar amounts of saline in the test culture with no LAE or DIMPTS. The test cultures were incubated at 37°C for 24 hr, the total viable bacteria were numerated by plate count on Trypticase® soy agar (BBL) containing inactivating agent. The plates were incubated at 37°C for 48 hr and reported as colony forming unit/ml (CFU/ml).

**Table 1. Synergistic effect of LAE with DIMPTS in vitro against Gram positive pathogens and fungi**

| | **CFU/ml** | | |
|---|---|---|---|
| **Treatment** | ***C. albican*** | ***S. aureus*** | ***E. faecium*** |
| Control | 5.3 x 10⁸ | 1.3 x 10⁹ | 8.4 x 10⁸ |
| LAE 10 ppm | 6x10⁷ | 1.1x10⁹ | 5x10⁷ |
| DIMPTS 0.5 ppm | 1 x10⁸ | 1.2 x 10⁹ | 6 x 10⁸ |
| DIMPTS 5 ppm | N/A | 2.4 x 10⁶ | 3 x 10⁸ |
| LAE/ DIMPTS 10/0.5 ppm | 300 | 1 x10⁸ | 2 x 10⁶ |
| LAE/ DIMPTS 10/5 ppm | N/A | <10 | 3 x 10³ |

**Table 2. Synergistic effect of LAE with DIMPTS in vitro against Gram negative pathogens**

| | **CFU/ml** | |
|---|---|---|
| **Treatment** | ***E. coli*** | ***P. aeruginosa*** |
| Control | 1.6 x 10⁹ | 1.3 x10⁹ |
| LAE 10 ppm | 1.1 x 10⁹ | 1.2 x10⁹ |
| DIMPTS 10 ppm | 1.4 x10⁹ | 1.0 x10⁹ |
| DIMPTS 200 ppm | 1.1 x10⁵ | 4 x10⁸ |
| LAE/ DIMPTS 10/10 ppm | 1.5 x10⁶ | 8.2 x10⁸ |
| LAE/ DIMPTS 10/200 ppm | <10 | 3 x10³ |

Results in Tables 1 and 2 show the synergistic activity of Lauric arginate (LAE) and diiodomethyl-p-tolylsulfone (DIMPTS).

### Example 2

### Antimicrobial efficacy of sutures coated with LAE

A series of USP standard size 2-0 uncoated polyglactin 910 sutures were coated with coating compositions containing LAE. LAE stock solution was made in ethanol at the concentration of 10%. The coating solutions were made by dissolving a L(-) lactide/glycolide copolymer containing 65 mole% lactide and 35 mole% glycolide (4.5%) and calcium stearate (4.5%) in ethyl acetate. Then, the LAE stock solution was added to the coating solution at sequential concentrations and mixed thoroughly. The sutures were hand coated by dipping into the coating solution containing antimicrobial composition and then air dried at room temperature for 8 hr. The antimicrobial efficacy was evaluated by zone of inhibition assay, in which the sutures were cut into a 5 cm section and inoculated with about 10⁵ cfu/suture in a Petri dish, a portion of 20 ml of TSA tempered at 47°C was added into the Petri dish. The inoculum was mix thoroughly with the medium and the suture was placed in the middle of the plate. The inoculated plates were incubated at 37°C for 48 hr and then the zone of inhibition was measured with a digital caliper.

**Table 3. Antimicrobial efficacy of sutures coated with LAE**

| % of LAE in coating solution | Zone of inhibition(mm) | | |
|---|---|---|---|
| | *S*. *aureus* | *E. coli* | *C. albicans* |
| Control | 0 | 0 | 0 |
| 0.5% LAE | 0 | 0 | 0 |
| 1% LAE | 1.5 | 1.0 | 2.3 |
| 2% LAE | 3.2 | 2.0 | 3.5 |

The zone of inhibition assay was performed against *S*. *aureus, E. coli* and *C*. *albicans* over a two-day period. The results in Table 3 indicate that sutures made with coating solutions containing equal or more than 1 % LAE demonstrated in vitro efficacy against *S*. *arueus C. albicans* and *E. coli.* The untreated suture exhibited no zones of inhibition. This result indicated the applicability of LAE as antimicrobial agents to be used in medical devices, such as sutures.

### Example 3

### Antimicrobial efficacy of suture coated with diiodomethyl-p-tolylsulfone (DIMPTS)

A series of USP standard size 2-0 uncoated polyglactin 910 sutures were coated with coating compositions containing DIMPTS. DIMPTS stock solution was made in ethanol at the concentration of 10%. The coating solutions were made by dissolving a L(-) lactide/glycolide copolymer containing 65 mole% lactide and 35 mole% glycolide (4.5%) and calcium stearate (4.5%) in ethyl acetate. Then, the DIMPTS stock solution was added to the coating solution at sequential concentrations and mixed thoroughly. The sutures were hand coated by dipping into the coating solution containing antimicrobial composition and then air dried at room temperature for 8 hr. The antimicrobial efficacy was evaluated by zone of inhibition assay, in which the sutures were cut into a 5 cm section and inoculated with about 10⁵ cfu/suture in a Petri dish, a portion of 20 ml of TSA tempered at 47°C was added into the Petri dish. The inoculum was mix thoroughly with the medium and the suture was placed in the middle of the plate. The inoculated plates were incubated at 37°C for 48 hr and then the zone of inhibition was measured with a digital caliper.

**Table 4. Antimicrobial efficacy of sutures coated with DIMPTS**

| % of DIMPTS in coating solution | Zone of inhibition (mm) | | |
|---|---|---|---|
| | *S*. *aureus* | *E. coli* | *C. albicans* |
| Control | 0 | 0 | 0 |
| 0.1 % DIMPTS | 0 | 0 | 3.3 |
| 0.5 % DIMPTS | 3.0 | 0 | 3.6 |
| 1% DIMPTS | 4.3 | 1.0 | 8.4 |

The zone of inhibition assay was performed against S. *aureus, E. coli and C*. *albicans* over a two-day period. The results in Table 4 indicate that sutures made with coating solutions containing equal or more than 0.1 % DIMPTS for *C*. *albicans,* and equal or more than 0.5% DIMPTS for *S*. *aureus and E. coli* demonstrated in vitro antimicrobial efficacy. The untreated suture exhibited no zones of inhibition. This result indicated the applicability of DIMPTS as antimicrobial agents to be used in medical devices, such as sutures.

### Example 4

### Synergy of sutures coated with LAE and diiodomethyl-p-tolylsulfone (DIMPTS)

The synergistic effect described in Example 1 is demonstrated with medical devices coated with combination of LAE and DIMPTS. A series of USP standard size 2-0 uncoated polyglactin 910 sutures were coated with coating compositions containing the combinations of LAE and DIMPTS. DIMPTS and LAE stock solutions were made in ethanol at concentration of 10%. The coating solutions were made by dissolving a L(-) lactide/glycolide copolymer containing 65 mole% lactide and 35 mole% glycolide (4.5%) and calcium stearate (4.5%) in ethyl acetate. Then, the DIMPTS and LAE stock solutions were added to the coating solution at sequential concentrations and mixed thoroughly. The sutures were hand coated by dipping into the coating solution containing antimicrobial composition and then air dried at room temperature for 8 hr. The antimicrobial efficacy and synergistic effect were evaluated by zone of inhibition assay, in which the sutures were cut into a 5 cm section and inoculated with about 10⁵ cfu/suture in a Petri dish, a portion of 20 ml of TSA tempered at 47°C was added into the Petri dish. The inoculum was mix thoroughly with the medium and the suture was placed in the middle of the plate. The inoculated plates were incubated at 37°C for 48 hr and then the zone of inhibition was measured with a digital caliper.

**Table 5. Synergistic effect of LAE and diiodomethyl-p-tolylsulfone (DIMPTS) in suture**

| % of LAE or/and DIMPTS in coating solution | Zone of inhibition (mm) | |
|---|---|---|
| | *S. aureus* | *E. coli* |
| Control | 0 | 0 |
| 0.5% LAE | 0 | 0 |
| 0.1 % DIMPTS | 0 | 0 |
| 0.5% LAE, 0.1 DIMPTS | 3.9 | 2.0 |

The zone of inhibition assay was performed against *S*. *aureus* and *E. coli* over a two-day period. The results, shown in Table 5, indicate that sutures made with LAE and DIMPTS demonstrated in vitro efficacy against *S*. *arueus* and *E. coli,* and the combination of lauric arginate (LAE) and DIMPTS resulted in superior antimicrobial activity against *S*. *aureus* and *E*. *coli*, compared to use LAE and DIMPTS individually. The untreated suture exhibited no zones of inhibition. This result indicated the applicability of LAE and DIMPTS in combination as a synergistic antimicrobial composition to be used in medical devices, such as sutures. Moreover, the synergistic effect observed could allow suture with good antimicrobial efficacy to be made with relatively lower concentration of LAE and DIMPTS then they were used alone. The reduced use of LAE and DIMPTS would have less impact to the physical properties of the suture.

## Claims

1. An antimicrobial composition comprising:
an effective amount of a cationic surfactant derived from the condensation of fatty acids and esterified dibasic amino acids, according to the following formula: 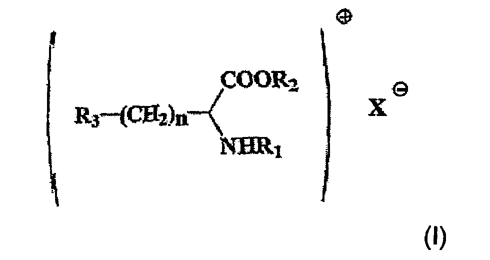 where X is Br, Cl, or HSO4; R₁ is a linear alkyl chain from a saturated fatty acid or hydroxyacid having from 8 to 14 carbon atoms bonded to the alpha-amino acid group through an amidic bond; R₂ is a linear or branched alkyl chain from 1 to 18 carbon atoms or an aromatic group; R₃ is one of the following:
-NH₃ 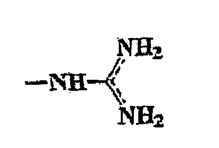 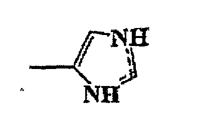
and n ranges from 0 to 4;
and at least one iodinated sulfone compound.

2. The antimicrobial composition of claim 1, wherein the cationic surfactant is lauric arginate, according to the following formula:

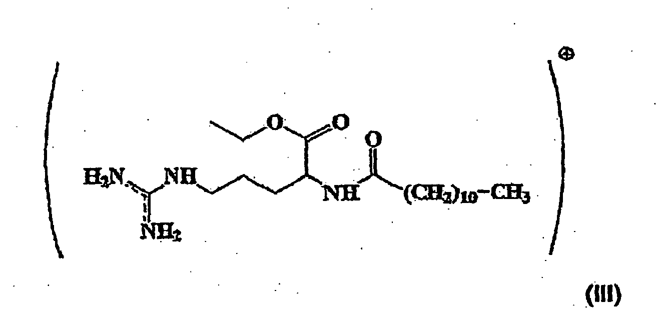

3. The antimicrobial composition of claim 1, wherein the iodinated sulfone compound.is diiodomethyl-p-tolylsulfone.

4. The antimicrobial composition of claim.3 wherein the concentration of said lauric arginate is between 0.001 % and 10% by weight based on the total weight, and of said diiodomethyl-p-tolylsulfone is between 0.001% and 5% by weight based on the total weight.

5. The antimicrobial composition of claim 3 wherein the concentration of said lauric arginate is between 0.01% and 2% by weight based on the total weight, and of said diiodomethyl-p-totylsulfone is between 0.01% and 1.5% by weight based on the total weight.

## Patentansprüche

1. Antimikrobielle Zusammensetzung, umfassend:
eine wirksame Menge eines kationischen Tensids, gewonnen aus der Kondensation von Fettsäuren und veresterten zweibasigen Aminosäuren, gemäß der folgenden Formel: 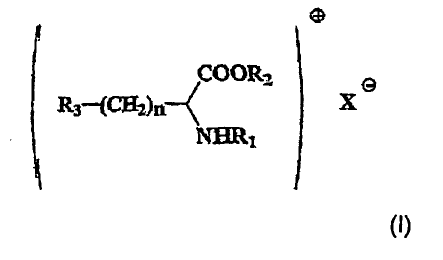 worin X Br, Cl oder HS04 ist; R₁ eine lineare Alkylkette aus einer gesättigten Fettsäure oder Hydroxysäure mit von 8 bis 14 Kohlenstoffatomen, gebunden an die alpha-Aminosäuregruppe durch eine Amidbindung, ist; R₂ eine lineare oder verzweigte Alkylkette von 1 bis 18 Kohlenstoffatomen oder eine aromatische Gruppe ist; R₃ eine der Folgenden ist:
—NH₃ 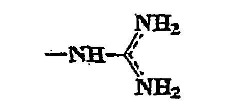 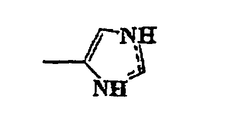
und n von 0 bis 4 reicht;
und wenigstens eine iodierte Sulfonverbindung.

2. Antimikrobielle Zusammensetzung nach Anspruch 1, wobei das kationische Tensid Laurinsäurearginat gemäß der folgenden Formel ist:

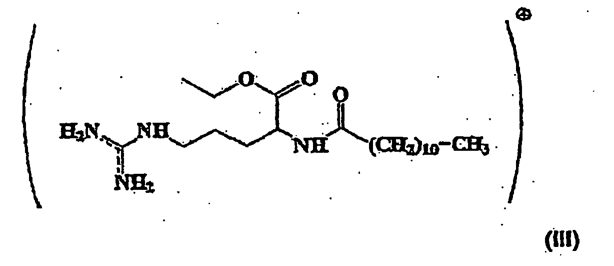

3. Antimikrobielle Zusammensetzung nach Anspruch 1, wobei die iodierte Sulfonverbindung Diiodmethyl-p-tolylsulfon ist.

4. Antimikrobielle Zusammensetzung nach Anspruch 3, wobei die Konzentration des Laurinsäurearginats zwischen 0,001 und 10 Gew.-%, bezogen auf das Gesamtgewicht, und des Diiodmethyl-p-tolylsulfons zwischen 0,001 und 5 Gew.-%, bezogen auf das Gesamtgewicht, beträgt.

5. Antimikrobielle Zusammensetzung nach Anspruch 3, wobei die Konzentration des Laurinsäurearginats zwischen 0,01 und 2 Gew.-%, bezogen auf das Gesamtgewicht, und des Diiodmethyl-p-tolylsulfons zwischen 0,01 und 1,5 Gew.-%, bezogen auf das Gesamtgewicht, beträgt.

## Revendications

1. Composition antimicrobienne comprenant :
une quantité efficace d'un tensioactif cationique dérivant de la condensation d'acides gras et d'acides aminés dibasiques estérifiés, conforme à la formule suivants : 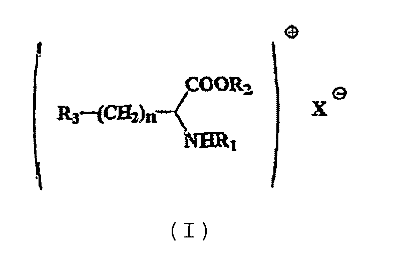 dans laquelle X est Br, Cl ou HSO₄ ; R₁ est une chaîne alkyle linéaire provenant d'un hydroxy-acide ou acide gras saturé ayant de 8 à 14 atomes de carbone, lié au groupe acide α-aminé par l'intermédiaire d'une liaison amide ; R₂ est une chaîne alkyle linéaire ou ramifiée ayant de 1 à 18 atomes de carbone ou un groupe aromatique ; R₃ est l'un des suivantes :
—NH₃ 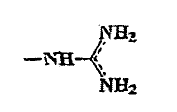 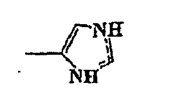
et n est situé dans la plage allant de 0 à 4 ;
et au moins un composé sulfone iodé.

2. Composition antimicrobienne selon la revendication 1, dans laquelle le tensioactif cationique est l'alginate laurique, conforme à la formule suivants :

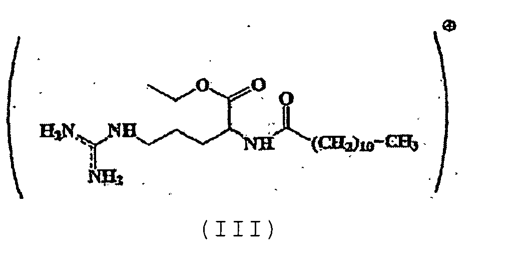

3. Composition antimicrobienne selon la revendication 1, dans laquelle le composé sulfone iodé est la diiodométhyl-p-tolylsulfone.

4. Composition antimicrobienne selon la revendication 3, dans laquelle la concentration dudit alginate laurique est comprise entre 0,001 % et 10 % en poids par rapport au poids total, et celle de ladite diiodométhyl-p-tolylsulfone est comprise entre 0,001 % et 5 % en poids par rapport au poids total.

5. Composition antimicrobienne selon la revendication 3, dans laquelle la concentration dudit alginate laurique est comprise entre 0,01 % et 2 % en poids par rapport au poids total, et celle de ladite diiodométhyl-p-tolylsulfone est comprise entre 0,01 % et 1,5 % en poids par rapport au poids total.
